# EUROPÄISCHE PATENTSCHRIFT

(11) **EP 0 489 937 B1**
(45) Veröffentlichungstag und Bekanntmachung des Hinweises auf die Patenterteilung: **21.06.1995**
(21) Anmeldenummer: 90123498.9
(22) Anmeldetag: 07.12.1990
(51) Int. Cl.: A61M 25/01, A61B 1/00

(54) **Medizinisches Instrument mit lenkbarer Spitze**
Medical instrument with steerable tip
Instrument médical avec tête orientable

(43) Veröffentlichungstag der Anmeldung: 17.06.1992
(73) Patentinhaber: WILLY RÜSCH AG, D-71385 Kernen (DE)
(72) Erfinder: Schmitt, Klaus, W-7064 Remshalden-Grunbach (DE); Entenmann, Helmut, W-7060 Schorndorf-Schornbach (DE)
(74) Vertreter: KOHLER SCHMID + PARTNER

(56) Entgegenhaltungen:
- EP-A- 0 165 718
- WO-A-90/07355
- DE-A- 3 214 615
- US-A- 3 605 725
- US-A- 4 586 923
- US-A- 4 686 963

## Beschreibung

Die Erfindung geht aus von einem medizinischen Instrument, bestehend aus einem relativ steifen Schaft und einer flexiblen Spitze, die an dem einen Ende des Schaftes in dessen Verlängerung angebracht ist und an deren äußerem Ende Zugseile befestigbar sind, die dicht unter den Oberflächendes Schaftes und der Spitze im wesentlichen parallel zur Spitze und zum Schaft bis an dessen anderes Ende in einem jeweils rohrförmigen Kanal geführt und am anderen Ende des Schaftes mit einem Betätigungsglied verbunden sind, das eine Verkürzung eines Zugseiles ermöglicht, um der Spitze eine wählbare Krümmung in Richtung des verkürzten Zugseiles zu erteilen, und bei dem der Schaft und die Spitze aus extrudierten Kunststoffteilen bestehen, die am Übergang vom Schaft zur Spitze miteinander verbunden sind, wobei das andere Ende des Schaftes in einem Handgriff befestigt ist.

Ein derartiger Katheter ist durch die US-A-3 605 725 bekannt geworden.

Das bekannte medizinische Instrument weist eine flexible Spitze auf, die durch mehrere parallel verlaufende Zugseile auslenkbar ist. Der Schaft des Instruments und die auslenkbare Spitze sind aus einem biegsamen Werkstoff gefertigt, wobei der des Schafts im Vergleich zu dem der Spitze weniger biegsam ist. Bei einer starken Auslenkung der Spitze wird auch der Schaft leicht gekrümmt, so daß die Steuerbarkeit der Spitze beeinträchtigt wird.

Derartige medizinische Instrumente sind in Form von Endoskopen mit "steuerbarer Spitze" bekannt. Der Schaft besteht aus einem relativ steifen Rohr aus Metall oder Kunststoff, dessen Inneres bei Endoskopen größeren Durchmessers als Instrumentenkanal, Spülkanal und dgl. eingerichtet ist, wogegen dünnere Endoskope zur besonderen Miniaturisierung ihres Querschnittes kein eigentliches Lumen mehr aufweisen, sondern lediglich von einem Strang optischer Fasern durchsetzt sind. Die flexible Spitze solcher Endoskope besteht aus einer Anzahl hintereinander angeordneter und gelenkig miteinander verbundener Glieder. An dem das äußere Ende der Spitze bildenden Glied sind gewöhnlich an zwei einander gegenüberliegenden Stellen die Seile zweier Bowdenzügen befestigt, deren Hüllen an den Außenseiten der Spitze und des Schaftes angebracht und insbesondere mittels einer die flexible Spitze und den Schaft sowie auch die Bowdenzüge umgebenden Hülle verbunden sind. Gelegentlich wird auch nur ein Bowdenzug verwendet, wenn darauf verzichtet werden kann, daß die Spitze auch mittels Bowdenzügen wieder gestreckt werden kann. Die Ausrichtung der gekrümmten Spitze läßt sich auch durch Drehen des Instrumentes um seine Längsachse erreichen. In jedem Fall ist die Ausbildung der bekannten medizinischen Instrumente, die an einem Ende eine steuerbare Spitze aufweisen, relativ kompliziert und erfordert einen Querschnitt erheblicher Größe, der ein solches Instrument für viele Anwendungszwecke ungeeignet macht.

Operationsmethoden, bei denen relativ dünne medizinische Instrumente in natürliche oder künstliche Körperkanäle eingeführt werden, gewinnen zunehmend an Bedeutung. Eine steuerbare Spitze ermöglicht es dabei, das Instrument in abzweigende oder im Bereich von Körperhöhlen versetzt weiterführende Kanäle gezielt einzuführen. Daher sind medizinische Instrumente mit gezielt steuerbarer Spitze für viele Anwendungszwecke geeignet und bieten dem Operateur nicht nur neue Möglichkeiten, sondern gewährleisten auch eine schonendere Behandlung des Patienten.

Bei dem bekannten medizinischen Instrument sind die Zugseile zur Steuerung der Spitze im Handgriff an einer Steuerplatte befestigt, die von außen über einen Hebel so verkippt werden kann, daß ein Zugseil gestrafft und die Spitze ausgelenkt wird. Der Anwender wird in der Regel mit der einen Hand den Handgriff des Instruments halten und mit der anderen Hand die Spitze steuern. Der Anwender hat daher bei der Bedienung des bekannten Instruments keine Hand frei, das Lumen des Schafts zu nutzen. Dies ist aber bei medizinischen Eingriffen besonders vorteilhaft, da es beispielsweise notwendig sein kann, einen Absaugschlauch einzuführen.

Der aus der DE-OS 14 91 697 bekannte Katheter weist einen Kunststoffschaft auf, der mit einer Spitze verschweißbar ist, die aus einem zum Schaft unterschiedlichen Kunststoff bestehen kann. Ferner ist der Schaft wie auch die Spitze mit einem Innenlumen versehen, das eine Biegevorrichtung aufnehmen kann. Die Steifigkeit des Katheters selbst wird durch unterschiedliche Geflechte erreicht, die in das Kunststoffschaftmaterial sowie in die Spitze eingearbeitet werden. Soll die Spitze aus der natürlichen Achse verschwenkt werden, so muß die Biegevorrichtung, die aus einem Führungsrohr und einem biegsamen Bowdenzug besteht, durch das Innenlumen bis in den Bereich der Spitze vorgeschoben werden. Aufgrund der Betätigung eines Hebels am körperfernen Ende des Katheters wird der Bowdenzug bewegt, und die Spitze des bekannten Katheters lenkt aus. Neben dem aufwendigen Aufbau der Biegevorrichtung ist es nachteilig, daß das zentrale Innenlumen von der Biegevorrichtung belegt ist und nicht für das Einschieben zusätzlicher Instrumente zur Verfügung steht. Ferner ist der Außendurchmesser wie auch der Durchmesser des Innenlumens vom Durchmesser der Biegevorrichtung abhängig.

Aus der US-A-4 686 963 und der US-A-4 586 923 sind medizinische Instrumente mit einer lenkbaren Spitze bekannt, die durch mehrere, über seitlich angebrachte Drehknöpfe spannbare Zugseile gesteuert werden kann.

Daher liegt der Erfindung die Aufgabe zugrunde, ein medizinisches Instrument der eingangs genannten Art zu vereinfachen und zugleich derart weiterzubilden, daß es auch noch mit extrem geringen Durchmessern herstellbar ist und daß die Steuerfähigkeit der auslenkbaren Spitze erhöht und die Handhabung erleichtert wird.

Die Aufgabe wird nach der Erfindung dadurch gelöst, daß im Bereich des Handgriffs die die Zugseile führenden Kanäle des Schaftes nach außen geöffnet sind, daß als Betätigungsglied ein zu beiden Seiten aus dem Handgriff herausragendes Stellrad um eine zur Schaftachse senkrechte Achse drehbar gelagert ist, daß die Enden der Zugseile am Stellrad befestigt sind und daß das Stellrad an seinem Umfang in einer Aussparung des Handgriffs gelagert ist und eine zentrale Aussparung aufweist, durch die der im Handgriff befestigte Abschnitt des Schaftes hindurchführbar ist.

Die erfindungsgemäße Ausbildung des medizinischen Instrumentes bietet die Möglichkeit, auf besonders einfache Weise mehrere Zugseile mit den für eine einwandfreie Steuerung der Spitze notwendigen Betätigungsgliedern zu verbinden.

Weist das Instrument wenigstens ein Paar einander gegenüberliegender Zugseile auf, so werden zweckmäßig die Enden eines Paares der Zugseile in zwei zur Richtung des Schaftes und zur Drehachse des Stellrades symmetrischen Stellen befestigt.

Diese Anordnung ermöglicht es, den Handgriff zu halten und gleichzeitig mit Daumen und Zeigefinger das aus dem Handgriff herausragende Stellrad an zwei einander gegenüberliegenden Stellen zu erfassen und dadurch leicht zu drehen. So läßt sich, bei entsprechender Befestigung des Schaftes in dem Handgriff, die flexible Spitze des Instrumentes in der durch das Handrad definierten Ebene abbiegen. Dadurch ist trotz der sehr einfachen Ausbildung des erfindungsgemäßen medizinischen Instrumentes auch dessen Handhabung sehr einfach. Es lassen sich bei Bedarf im Handgriff auch zwei Stellräder unterbringen, um daran die Zugseile zweier Paare zu befestigen, die das Steuern der Spitze in zwei zueinander senkrechten Ebenen ermöglichen.

Das Stellrad ist an seinem Umfang in einer Aussparung des Handgriffes gelagert und weist eine zentrale Aussparung auf, durch die der im Handgriff befestigte Abschnitt des Schaftes hindurchgeführt ist. Diese Ausbildung des Stellrades ist dann von besonderem Vorteil, wenn der Schaft des Instrumentes ein zentrales Lumen aufweist, das vom Ende des Handgriffes her zugänglich sein soll. Die vorliegende Erfindung ermöglicht es, den Schaft gänzlich durch den Handgriff hindurchzuführen oder aber das im Schaft vorhandene Lumen durch eine im Handgriff vorgesehene Bohrung fortzusetzen. Gleichzeitig ist dadurch die Möglichkeit gegeben, den Schaft auf einer verhältnismäßig großen Länge im Handgriff einzuspannen, woraus eine sehr sichere Befestigung resultiert. Der Schaft kann auch unterhalb des Stellrades durch den Handgriff hindurchgeführt werden. Am Stellrad kann auch noch ein Hebel mit Fingerring angebracht sein. Dies ist dann zweckmäßig und besonders vorteilhaft, wenn am Handgriff noch ein zusätzlicher Griffabschnitt ausgebildet ist, damit das Instrument pistolenartig gehalten und die Spitze durch eine Vorwärts-und Rückwärtsbewegung des Fingers mittels eines Eingriffes des Fingers in den Ring am Stellrad definiert und leicht ausgelenkt werden kann.

In weiterer Ausgestaltung der Erfindung kann das Stellrad aus zwei scheibenförmigen Teilen bestehen, die an ihren einander zugewandten Seiten Vertiefungen bzw. Vorsprünge aufweisen, welche die Aussparungen für den Schaft und Führungsflächen für das aus dem Schaft herausgeführte Ende des Zugseiles bilden, und es kann das Ende des Zugseiles zwischen aneinanderliegenden Flächenabschnitten der scheibenförmigen Teile eingeklemmt sein. Diese Ausbildung des Stellrades fördert weiter die Einfachheit des Aufbaues des medizinischen Instrumentes und ermöglicht darüberhinaus eine einfache Montage.

Bei dem erfindungsgemäßen medizinischen Instrument sind also mehrere rohrförmige Kanäle für Zugseile in die den Schaft und die Spitze des Instrumentes bildenden Kunststoffteile integriert und werden unmittelbar bei der Herstellung dieser Kunststoffteile durch Extrudieren erzeugt. Die stumpfe Verbindung der aus Materialien unterschiedlicher Steifigkeit bestehenden Teile, also des Schaftes und der Spitze, kann auf verschiedene Weise erfolgen, insbesondere durch einfaches Verkleben oder Verschweißen. Nicht nur die Tatsache, daß es gelingt, Kunststoffstränge mit exzentrischen, äußerst engen Kanälen als Hüllen für die Zugseile herzustellen, muß als überraschend angesehen werden, sondern es war auch keineswegs ohne weiteres vorhersehbar, daß die den Schaft und die Spitze bildenden Kunststoffteile stumpf in solcher Weise miteinander verbunden werden können, daß diese Kanäle genau miteinander fluchten und nicht beim Herstellen der Verbindung verstopft werden. Eine solche Verbindung kann außerdem ausreichend fest gestaltet werden, um allen bei der Benutzung eines solchen Instrumentes auftretenden Belastungen standzuhalten, so daß keine Gefahr eines Loslösens der Spitze und demgemäß einer Schädigung des Patienten durch eine abgelöste und dann nicht mehr ohne weiteres entfernbare Spitze besteht.

In weiteren Ausgestaltungen der Erfindung sind die Zugseile im Bereich des Schaftes innerhalb des Kanals von einer Hülse ummantelt. Dies hat den Vorteil, daß die Spitzensteuerung noch weiter verbessert werden kann. Die Hülsen, aus Metall oder Kunststoff, versteifen den Schaft über seine gesamte Länge zusätzlich und halten diesen richtungsstabil, wenn die Spitze des erfindungsgemäßen Katheters verschwenkt wird. Eine möglicherweise eintretende Verbiegung des Schaftes beim Verschwenkvorgang der Spitze durch eine Streckenveränderung der Zugseile wird dadurch weiter eingeschränkt. Die Spitze selbst ist durch diese Maßnahme noch besser steuerbar.

Bei nach der Erfindung ausgebildeten medizinischen Instrumenten kann es sich insbesondere um Sonden beliebiger Art, um Katheter u.dgl. handeln. Auch bei diesen Instrumenten können der Schaft und die Spitze ein zentrales Lumen aufweisen, das als Absaugund/oder Spülkanal aber auch zum Einführen anderer Instrumente, insbesondere von Lichtleitern, Ultraschallgebern für die Lithotripsie u. dgl. dienen kann. Es können auch Lichtleiter, Schallgeber u. dgl. Instrumente fest in die Spitze eines solchen Instrumentes eingearbeitet sein. Ein weiteres Beispiel für ein medizinisches Instrument, das nach der Erfindung ausgebildet sein kann, ist eine Sonde für die Angioplastie, die im Bereich der Spitze einen expandierbaren Ballon aufweist. Gerade solche Sonden haben einen sehr geringen Durchmesser, und es ist äußerst vorteilhaft, daß es die Erfindung ermöglicht, auch solche Sonden mit einer gezielt steuerbaren Spitze zu versehen, welche das Einführen der Sonde in abzweigende Gefäße ermöglicht.

Für die Befestigung der Zugseile am äußeren Ende der Spitze stehen viele Möglichkeiten zur Verfügung, insbesondere Einkleben, Einpressen oder Einschmelzen. Die Anordnung muß allerdings so getroffen sein, daß keine Gefahr eines Abreißens der Zugseile besteht, wodurch die Lenkung der Spitze unmöglich würde. Eine besonders sichere Art der Befestigung wird erzielt, wenn in weiterer Ausgestaltung der Erfindung in dem äußeren Ende der Spitze ein Ring angeordnet ist, an dem die Zugseile befestigt sind. Eine andere Möglichkeit eröffnet sich dann, wenn an dem äußeren Ende der Spitze wenigstens ein Paar einander gegenüberliegender Zugseile befestigt ist. Dann können nämlich die beiden Zugseile des Paares von zwei Abschnitten eines durchgehenden Seiles gebildet werden, dessen die beiden Zugseile verbindendes Stück am Ende der Spitze in das die Spitze bildende Material eingeschmolzen ist. Auch die Verwendung eines durchgehenden Zugseiles gewährleistet eine praktisch unlösbare Verbindung der Zugseile mit dem Ende der flexiblen Spitze. Wenn der Schaft und die Spitze ein zentrales Lumen aufweisen, kann das in das Ende der Spitze eingeschmolzene Stück des Seiles eine das Lumen umgebende Windung bilden. Auf diese Weise wird das Lumen frei gehalten und durch die Windung zugleich eine sehr großflächige und damit sehr sichere Befestigung des Seiles am Ende der Spitze erzielt. Ebenfalls kann die Spitze mit einer Öffnung versehen werden, die mit dem zentralen Lumen verbunden ist.

Es ist ersichtlich, daß das dargestellte Instrument sehr einfach aufgebaut ist. Der Schaft und die Spitze sind durch Extrudieren leicht herstellbar und die einzelnen Teile sind auch auf einfache Weise zusammenfügbar. Die steuerbare Spitze dieses Katheters eröffnet viele neue Möglichkeiten, insbesondere auch bei der Lithotripsie, weil die Spitze des Instrumentes, durch dessen Lumen ein Lichtleiter oder auch ein Ultraschall-Geber eingeführt und genau auf einen zu zerstörenden Stein aufgesetzt werden kann.

Es versteht sich, daß die Erfindung nicht auf das dargestellte Ausführungsbeispiel beschränkt ist, sondern Abweichungen davon möglich sind, ohne den Rahmen der Erfindung zu verlassen. Insbesondere ist ohne weiteres erkennbar, daß ein solches Instrument mit zwei Paaren von Zugdrähten versehen werden kann, die in zwei zueinander senkrechten Ebenen angeordnet und jeweils mit einem eigenen Stellrad verbunden sind, so daß das Ende der flexiblen Spitze in jede beliebige Richtung gelenkt werden kann.

Für das Ausrichten der Spitze des Instrumentes auf eine bestimmte Stelle ist es von Vorteil, wenn diese Spitze für Röntgenstrahlen undurchlässig ist, so daß ihre Lage auf dem Röntgenschirm beobachtet werden kann. Für sehr empfindliche Einstellungen wäre sogar die Verwendung von drei Zugseilpaaren denkbar.

Es ist weiterhin ohne weiteres ersichtlich, daß die Erfindung bei medizinischen Instrumenten verwirklicht werden kann, die für eine Vielzahl unterschiedlicher Anwendungen speziell ausgebildet sind. Dabei kann es sich um Sonden ohne zentrales Lumen, aber auch um Instrumente mit mehreren Lumen handeln, die als Kanäle für andere Instrumente, zum Absaugen, zum Spülen, zum Expandieren eines an der Spitze des Instrumentes angebrachten Ballons u. dgl. dienen können. So kann ein solches Instrument insbesondere als Sonde für die Angioplastie Verwendung finden, bei der die gezielt steuerbare Spitze auch das Einführen der Sonde in von einem Hauptgefäß abzweigende Gefäßen gestattet, was bisher nicht möglich war. Daher eröffnet die Erfindung in vielen Anwendungsbereichen neue und vorteilhafte Möglichkeiten zur Untersuchung und Behandlung sehr unterschiedlicher Affektionen.

In weiterer Ausgestaltung der Erfindung ist am Handgriff ein Seitenkanal vorgesehen, der mit dem Lumen des Katheters in Verbindung steht. Über diesen Kanal können gezielt eine Flüssigkeit bzw. Gase oder Aerosole in Körperhöhlungen eingebracht werden. Wird über das zentrale Lumen des erfindungsgemäßen Katheters ein zusätzliches Intstrument, wie eine Optik, beispielsweise aus Glasfasern oder ein Greifwerkzeug eingeführt, so kann über den Seitenkanal (sideport) ein Spülstrom verabreicht werden. Dies ist dann besonders vorteilhaft, wenn desintegrierte Steinfragmente aus einer Körperhöhlung auszuspülen sind (Laser-Lithotripsie). In blutführenden Gefäßen, aber auch in der Gallenblase und in ihrem Hohlsystem sowie bei perforierten Gängen (Speichelgang, Gallengang) kann ein Spülstrom von Kochsalzlösung für freie Sicht mittels eines Endoskops sorgen. Die Gefäße wie auch Gefäßanastomosen können optisch besser inspiziert werden.

Werden in einer weiteren Ausführungsform der Erfindung in das Kunststoffmateril der Spitze Bänder eingearbeitet, die sich mit einer schmalen Seite symmetrisch und quer zur Achse des Schaftes gegenüberliegen und mit der schmalen Seite zum Lumen hinweisen, wobei die Bänder zu den Zugseilen jeweils um 90° versetzt sind, so wird der Schaft wie auch die Spitze noch weiter zwangsstabilisiert. Die Spitze läßt sich dann nur noch in einer Ebene verschwenken.

In weiterer Ausgestaltung der Erfindung ist in das Kunststoffmaterial des Schaftes ein Geflecht eingearbeitet, das dem erfindungsgemäßen Instrument eine hohe Torsionsstabilität verleiht und eine zielgenaue Ausrichtung der Spitze auch bei Drehbewegungen ermöglicht.

Die erwähnten Ausführungsformen sind nicht als abschließende Aufzählung zu verstehen, sondern haben vielmehr beispielhaften Charakter.

Die Erfindung ist in der Zeichnung dargestellt und wird anhand von Ausführungsbeispielen anhand der Zeichnung näher erläutert. Es zeigen:
- Fig. 1: die Seitenansicht eines medizinischen Instrumentes nach der Erfindung,
- Fig. 2: eine Seitenansicht des den Handgriff umfassenden Abschnittes des Instrumentes nach Fig. 1 in Richtung des Pfeiles II,
- Fig. 3: einen Schnitt längs der Linie III - III durch die Anordnung nach Fig. 2,
- Fig. 4: einen Schnitt längs der Linie IV - IV durch die Anordnung nach Fig. 2,
- Fig. 5: einen Schnitt längs der Linie V - V durch das Instrument nach Fig. 1,
- Fig. 6: die Spitze des Instrumentes nach Fig. 1 in vergrößertem Maßstab und in gekrümmtem Zustand,
- Fig. 7: einen Schnitt längs der Linie VII-VII der Fig. 6,
- Fig. 8: eine weitere Ausführungsform im Schnitt, der dem Schnitt der Fig. 7 entspricht;
- Fig. 8a: einen Schnitt VIIIa - VIIIa der Fig. 6, und
- Fig. 9: ein erfindungsgemäßes Instrument mit einem am Handgriffende angekoppelten Ansatzstück mit Seitenkanal und einem Verbindungskanal.

Die einzelnen Figuren der Zeichnung zeigen teilweise stark schematisiert den erfindungsgemäßen Gegenstand und sind nicht maßstäblich zu verstehen. Die Gegenstände der einzelnen Figuren sind teilweise stark vergrößert dargestellt, damit ihr Aufbau besser gezeigt werden kann.

Bei dem in der Zeichnung dargestellten medizinischen Instrument handelt es sich um einen lenkbaren Instrument, das einen Schaft 1 in Form eines relativ starren Katheterrohres mit zentralem Lumen 2 umfaßt, an dessen einem Ende eine flexible Spitze 3 befestigt ist, wogegen sein anderes Ende in einem Handgriff 4 befestigt ist.

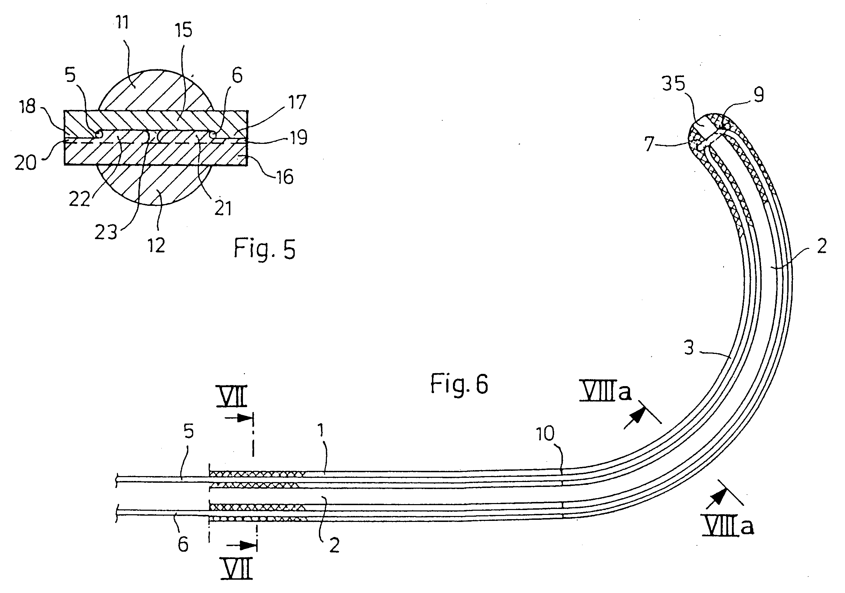

Wie aus Fig. 6 ersichtlich, wird durch die flexible Spitze 3 der Schaft 1 verlängert und auch das Lumen 2 des Schaftes 1 bis zum äußeren Ende 7 der Spitze 3 fortgesetzt. Weiterhin weisen der Schaft 1 und die Spitze 3 in ihrer Wandung an zwei einander gegenüberliegenden Stellen Kanäle auf, die Führungen für Zugseile 5, 6 bilden. Die Zugseile 5, 6 sind einerseits mit dem äußeren Ende 7 der Spitze 3 verbunden und andererseits in noch zu beschreibender Weise an einem im Handgriff 4 drehbar gelagerten Stellrad 8 befestigt. Die beiden Zugseile 5, 6 sind Abschnitte eines gemeinsamen, durchgehenden Seiles, dessen die beiden Zugseile 5, 6 verbindendes Stück 9 eine das Lumen 2 umgebende Windung bildet, die in das Ende 7 der Spitze 3 eingeschmolzen ist. Auf diese Weise wird eine sehr sichere Verbindung zwischen den Zugseilen 5, 6 und dem Ende 7 der Spitze 3 geschaffen, ohne daß durch die Befestigung der Zugseile 5, 6 das Lumen 2 verengt wird.

Der relativ steife Schaft 1 und die flexible Spitze 3 werden von extrudierten Kunststoffteilen gebildet, die am Übergang 10 vom Schaft 1 auf die Spitze 3 stumpf miteinander verklebt sind. Bei der Verklebung wurde darauf geachtet, daß nicht nur das Lumen 2, sondern auch die die Zugseile 5, 6 aufnehmenden Kanäle in den Wandungen des Schaftes 1 und der Spitze 3 genau miteinander fluchten und nicht durch Klebstoff eingeengt oder gar verstopft werden.

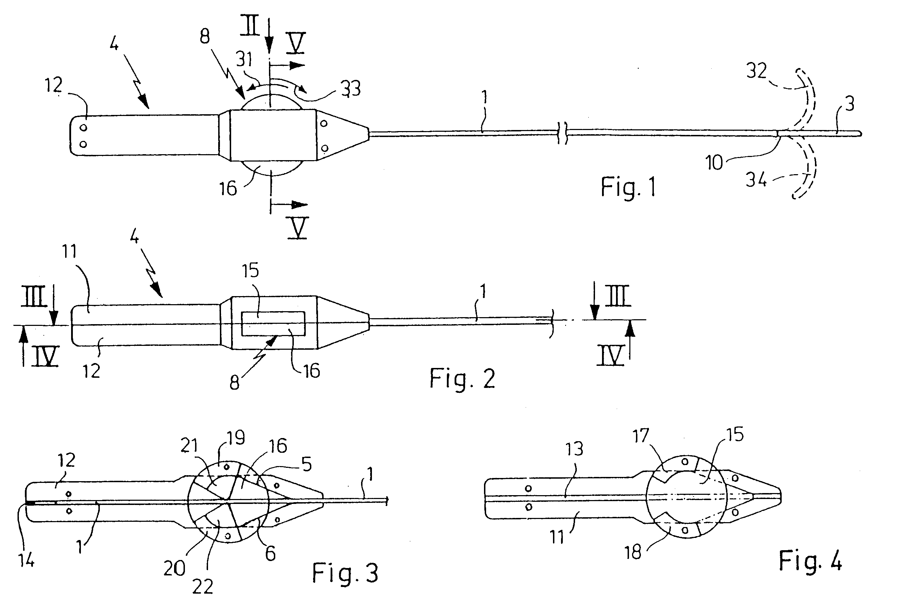

Der Handgriff 4 besteht aus zwei Halbschalen 11, 12, die eine zentrale Nut 13 bzw. 14 zur Aufnahme des Schaftes 1 aufweisen. Wenn die beiden Halbschalen 11, 12 zum Handgriff vereinigt sind, z.B. durch nicht näher dargestellte Schrauben miteinander verbunden sind, ist der Schaft 1 in der von den Nuten 13, 14 gebildeten Bohrung fest eingespannt. Das Stellrad 8 ist in entspreche Aussparungen der Halbschalen 11, 12 des Handgriffes 4 eingelegt und darin an seinem Umfang geführt. Das Stellrad 8 besteht aus zwei scheibenförmigen Teilen 15, 16, die bei montiertem Instrument fest miteinander verbunden sind, z.B. mittels nicht näher dargestellter Schrauben. Die scheibenförmigen Teile 15 und 16 liegen dabei mit im Bereich ihres Randes angeordneten Flächenabschnitten 17, 18 bzw. 19, 20 aneinander an. Im übrigen sind die einander zugewandten Flächen der beiden scheibenförmigen Teile 15, 16 gegenüber den Flächenabschnitten 17, 18, die Abschnitte eines Kreisringes bilden, so weit vertieft, daß der Schaft 1 des Instrumentes das von den beiden Teilen gebildete Stellrad 8 mit Spiel durchdringen kann. Im Axialverlauf des Stellrades 8 verläuft der Schaft 1 nicht in einer Nut oder Bohrung, sondern grenzt nur punktförmig an die Spitzen von sektorförmigen Abschnitten 21, 22. Die Spitzen sind beabstandet und der Zwischenraum dient dem Schaft 1 als zentrale Aussparung 23 (Fig. 5). Die an dem scheibenförmigen Teil 16 angebrachten sektorförmigen Abschnitte 21, 22, die noch über die Flächenabschnitte 19, 20 überstehen, kommen an der Innenfläche des gegenüberliegenden Teiles 15 zur Anlage. Diese sektorförmigen Abschnitte 21, 22 haben auf einem Kreis liegende äußere Ränder, an denen die Enden der Zugseile 5, 6 zur Anlage kommen, wenn sie zwischen den aneinanderanliegenden Flächen 17, 18 bzw. 19, 20 der scheibenförmigen Teile 15, 16 des Stellrades 8 eingespannt werden. Die Lage der Zugseile 5, 6, die aus entsprechenden seitlichen Öffnungen des Schaftes 1 herausgeführt sind, ist in Fig. 3 dargestellt.

Es ist ohne weiteres ersichtlich, daß bei einem Drehen am Stellrad 8 auf das eine der Zugseile 5, 6 ein Zug ausgeübt wird, während zugleich das andere Zugseil 6 bzw. 5 freigegeben wird, mit dem Ergebnis, daß die flexible Spitze 3 am Ende des Schaftes 1 nach der einen oder anderen Seite abgebogen wird, wie es in Fig. 1 gestrichtelt dargestellt ist. Dabei ist die Anordnung so getroffen, daß das Abbiegen der Spitze 3 in der gleichen Ebene stattfindet, in der sich das Stellrad 8 befindet, und auch nach der Seite hin, nach der das Stellrad 8 von der Spitze 3 weggedreht wird. Das heißt, daß bei der in Fig. 1 durch den Pfeil 31 angegebenen, entgegen dem Uhrzeigersinn gerichteten Drehung die Spitze 3 nach oben abgebogen wird, wie es die gestrichelte Kontur 32 angibt, wogegen bei einer Drehung in entgegengesetzter Richtung, nämlich in Richtung des Pfeiles 33, die Spitze 3 in die gestrichelt dargestellte Lage 34 gebracht wird.

In der Fig. 6 ist die Spitze 3 mit einer Öffnung 35 versehen. Durch die Öffnung 35 können Flüssigkeiten unmittelbar in eine Körperhöhlung eingebracht werden, oder ein weiteres Instrument läßt sich an diese Öffnung 35 durch das Lumen 2 heranführen.

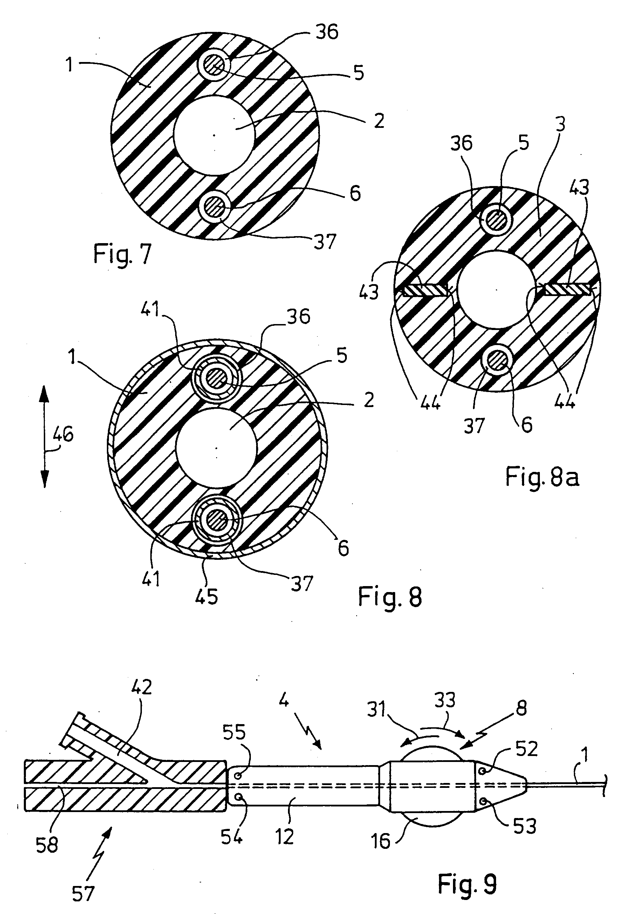

Fig. 7 zeigt einen Schnitt VII-VII der Fig. 6. Der Schaft 1 weist neben dem Lumen 2 einen Kanal 36 und 37 auf, in dem die Zugseile 5, 6 geführt sind. Der Schaft 1 selbst ist aus einem elastischen Kunststoff gefertigt.

Fig. 8 zeigt eine weitere Ausführungsform im Schnitt, wie der Schaft 1 bzw. die Spitze 3 aufgebaut sein kann. Der Schaft 1 ist in der Fig. 8 ebenfalls mit Kanälen 36, 37 versehen, die eine Hülse 41 aus einem biegestabilen Material, wie Metall oder hartem Kunststoff, aufnehmen. Die Hülsen 41 enden im Schaft 1 am Übergang zur Spitze 3. In der Hülse 41 ist das Zugseil 5 bzw. 6 geführt. Die Hülse 41 stabilisiert den Schaft 1 gegen Zugbewegungen und erhöht zugleich die Biegestreifigkeit des Schaftes 1. Der in der Figur gezeigte Schaft 1 ist noch mit einem Geflecht 45 ummantelt, das den Schaft 1 zusätzlich versteift und ihm eine hohe Torsionsstabilität verleiht.

Fig. 8a zeigt einen Querschnitt gemäß dem Schnitt VIIIa - VIIIa der Figur 6. In den Schaftabschnitt der Spitze 3 sind Bänder 43 eingearbeitet. Bei den Bändern 43 kann es sich um Metallbänder bzw. Gewebebänder oder aber auch Kunststoffbänder handeln. Die Bänder 43 stablisieren die Spitze 3 und wirken Auslenkungen der Spitze 3 zu einer schmalen Seite 44 der Bänder 43 entgegen.

In der gezeigten Ausführungsform kann sich die Spitze 3 des Schaftes 1 nur in Pfeilrichtungen 46 bewegen. Die Bänder 43 verhindern eine Bewegung quer zur Pfeilrichtung 46. Auch im Schaftabschnitt der Spitze 3 verlaufen die Zugseile 5, 6 in Kanälen 36, 37.

Der Außendurchmesser des Schaftes 1 und der Spitze 3 ist minimal ca. 1,5 mm. Das Lumen 2 weist dabei einen Durchmesser von ca. 0,7 mm auf. Die Kanäle 36, 37 haben einen Durchmesser von jeweils 0,25 mm bis 0,3 mm. Die Bänder 43 werden bevorzugt mit einer Breite von 0,3 mm und einer Höhe von 0,05 mm verwendet. Die Zugseile 5, 6 sind Edelstahllitzen mit einem Durchmesser von ca. 0,15 mm.

Fig. 9 zeigt den Schaft 1 mit dem Handgriff 4, wie er auch in der Fig. 1 dargestellt ist. Zusätzlich zu dem in der Fig. 1 dargestellten medizinischen Instrument ist an das Ende des Handgriffes 4 ein Ansatzstück 57 angekoppelt, das einen Verbindungskanal 58 aufweist, der mit dem Lumen des Schaftes 1 verbunden ist. Der Schaft 1 ist, wie mit gestrichelten Linien in der Figur gezeigt, durch den Handgriff 4 hindurchgeführt und ist mit dem Verbindungskanal 58 trennbar verbunden. Drehbewegungen des Stellrades 8 beeinflussen nicht die Lage des Schaftes 1 im Handgriff 4.

Der Schaft 1 ist in der Fig. 9 ohne lenkbare Spitze gezeigt. Die Spitze ist über das zweiteilige Stellrad 8, von dem in der Figur das scheibenförmige Teil 16 zu sehen ist, in Richtung der Pfeile 31, 33 verschwenkbar. Der Handgriff 4 ist ebenfalls zweiteilig aufgebaut und wird über Verbindungselemente 52, 53, 54, 55, wie Schrauben, zusammengehalten. Der Handgriff 4 ist an seinem von dem Schaft 1 wegweisenden Ende mit einem Verbindungselement versehen, über das das Ansatzstück 57 an den Handgriff 4 ankoppelbar ist. Das Ansatzstück 57 weist auch einen Seitenkanal 42 auf, der mit herkömmlichen Spritzensystemen verbindbar ist. Über den Seitenkanal 42 können dem Lumen des Schaftes 1 eine Flüssigkeit bzw. Gase oder Aerosole zugeführt werden. Der Seitenkanal 42 kann an seinem freien Ende ein in der Figur nicht gezeigtes Ventil oder einen Hahn aufweisen. Im Bereich des offenen Ende des Verbindungskanals 58 kann im Verbindungskanal 58 selbst und/oder im Seitenkanal 42 noch ein Septum vorgesehen sein, das gewährleistet, daß eine über den Seitenkanal 42 zugeführte Flüssigkeit nur in das Lumen des Schaftes 1 und durch den Handgriff 4 strömen kann. Durch den Verbindungskanal 58 können in das Lumen des Schaftes 1 beispielsweise ein Greifwerkzeug oder eine Glasfaseroptik eingeführt werden.

## Patentansprüche

1. Medizinisches Instrument, bestehend aus einem relativ steifen Schaft (1) und einer flexiblen Spitze (3), die an dem einen Ende des Schaftes (1) in dessen Verlängerung angebracht ist und an deren äußerem Ende (7) Zugseile (5, 6) befestigbar sind, die dicht unter den Oberflächen des Schaftes (1) und der Spitze (3) im wesentlichen parallel zur Spitze (3) und zum Schaft (1) bis an dessen anderes Ende in jeweils einem rohrförmigen Kanal geführt und am anderen Ende des Schaftes (1) mit einem Betätigungsglied verbunden sind, das eine Verkürzung eines Zugseiles (5, 6) ermöglicht, um der Spitze (3) eine wählbare Krümmung in Richtung des verkürzten Zugseiles (5, 6) zu erteilen, und bei dem der Schaft (1) und die Spitze (3) aus extrudierten Kunststoffteilen bestehen, die am Übergang (10) vom Schaft (1) zur Spitze (3) miteinander verbunden sind, wobei das andere Ende des Schafts (1) in einem Handgriff (4) befestigt ist, dadurch gekennzeichnet, daß im Bereich des Handgriffs (4) die die Zugseile (5, 6) führenden Kanäle des Schaftes (1) nach außen geöffnet sind, als Betätigungsglied ein zu beiden Seiten aus dem Handgriff (4) herausragendes Stellrad (8) um eine zur Achse des im Handgriff (4) befestigten Abschnittes des Schaftes (1) senkrechte Drehachse drehbar gelagert ist, die Enden der Zugseile (5, 6) am Stellrad (8) befestigt sind und daß das Stellrad (8) an seinem Umfang in einer Aussparung des Handgriffes (4) gelagert ist und eine zentrale Aussparung (23) aufweist, durch die der im Handgriff (4) befestigte Abschnitt des Schaftes (1) hindurchführbar ist.

2. Medizinisches Instrument nach Anspruch 1, dadurch gekennzeichnet, daß das Stellrad (8) aus zwei scheibenförmigen Teilen (15, 16) besteht, die an ihren einander zugewandten Seiten Vertiefungen bzw. Vorsprünge (17 bis 22) aufweisen, welche die Aussparung für den Schaft (1) und die Führungsflächen für das aus dem Schaft herausgeführte Ende des Zugseiles (5, 6) bilden und daß das Ende des Zugseiles (5, 6) zwischen aneinanderliegenden Flächenabschnitten (17 bis 19) der scheibenförmigen Teile (15, 16) eingeklemmt ist.

3. Medizinisches Instrument nach Anspruch 1 oder 2, dadurch gekennzeichnet, daß die Zugseile (5, 6) im Bereich des Schaftes (1) innerhalb des Kanals von einer Hülse (41) ummantelt sind.

4. Medizinisches Instrument nach Anspruch 1 bis 3, dadurch gekennzeichnet, daß in dem äußeren Ende (7) der Spitze (3) ein Ring angeordnet ist, an dem die Zugseile (5, 6) befestigt sind.

5. Medizinisches Instrument nach einem der Ansprüche 1 bis 4, dadurch gekennzeichnet, daß die Zugseile (5, 6) von zwei Abschnitten eines durchgehenden Seiles gebildet werden, dessen die beiden Zugseile (5, 6) verbindendes Stück (9) am Ende (7) der Spitze (3) in das die Spitze (3) bildende Kunststoffmaterial eingeschmolzen ist.

6. Medizinisches Instrument nach einem der Ansprüche 1 bis 5, dadurch gekennzeichnet, daß der Schaft (1) und die Spitze (3) ein zentrales Lumen (2) aufweisen und daß das in das Ende (7) der Spitze (3) eingeschmolzene Stück (9) des Seiles eine das Lumen (2) umgebende Windung bildet.

7. Medizinisches Instrument nach einem der Ansprüche 1 bis 6, dadurch gekennzeichnet, daß die Enden eines Paares einander gegenüberliegender Zugseile (5, 6) an zwei zur Richtung des Schaftes (1) und zur Drehachse des Stellrades (8) symmetrischen Stellen des Stellrades (8) befestigt sind.

8. Medinzinisches Instrument nach einem der Ansprüche 1 bis 7, dadurch gekennzeichnet, daß am Handgriff (4) ein Seitenkanal (42) vorgesehen ist, der mit dem Lumen (2) in Verbindung steht.

9. Medizinisches Instrument nach einem der Ansprüche 1 bis 8, dadurch gekennzeichnet, daß in das Kunststoffmaterial der Spitze (3) Bänder (43) eingearbeitet sind, die sich mit einer schmalen Seite (44) symmetrisch und quer zur Achse des Schaftes (1) gegenüberliegen und mit der schmalen Seite (44) zum Lumen (2) hinweisen, wobei die Bänder (43) zu den Zugseilen (5, 6) jeweils um 90° versetzt sind.

10. Medizinisches Instrument nach einem der Ansprüche 1 bis 9, dadurch gekennzeichnet, daß in das Kunststoffmaterial des Schaftes (1) ein Geflecht (45) eingearbeitet ist.

## Claims

1. Medical instrument consisting of a relatively rigid shaft (1) and a flexible tip (3), which is attached to one end of the shaft (1) as an extension thereof and at whose outer end (7) tension cables (5, 6) can be fastened which are guided, in each case in a tubular conduit and immediately below the surfaces of the shaft (1) and the tip (3), essentially parallel to the tip (3) and the shaft (1), up to its other end and are joined, at the other end of the shaft (1), to an actuation element that allows a shortening of a tension cable (5, 6) so as to impart to the tip (3) a selectable curvature in the direction towards the shortened tension cable (5, 6), and the shaft (1) and the tip (3) consist of extruded plastic parts that are joined to one another at the transition (10) from the shaft (1) to the tip (3), whereby the other end of the shaft (1) is fastened to a handle (4), characterized in that in the area of the handle (4) the conduits of the shaft (1) guiding the tension cables (5, 6) open outwardly, an adjusting wheel (8), functioning as an actuation element and protruding from both sides of the handle (4), is rotatably supported about an axis of rotation which is perpendicular to the axis of the section of the shaft (1) mounted in the handle (4), the ends of the tension cables (5, 6) being fastened to the adjusting wheel (8), and the adjusting wheel (8) is supported at its periphery in a recess of the handle (4) and comprises a central recess (23) through which the section of the shaft (1) which is fastened to the handle (4) can be guided.

2. Medical instrument according to claim 1, characterized in that the adjusting wheel (8) consists of two disc-shaped parts (15, 16) that have depressions and projections, respectively, (17 to 22) on their sides facing one another, which constitute the recess for the shaft (1) and the guide surfaces for the end of the tension cable (5, 6) guided out of the shaft, and wherein the end of the tension cable (5, 6) is clamped between adjacent surface sections (17 to 19) of the disc-shaped parts (15, 16).

3. Medical instrument according to claim 1 or 2, characterized in that in the region of the shaft (1) inside the conduit, the tension cables (5, 6) are encased in a sleeve (41).

4. Medical instrument according to any one of claims 1 to 3, characterized in that a ring to which the tension cables (5, 6) are fastened is located in the outer end (7) of the tip (3).

5. Medical instrument according to any one of claims 1 to 4, characterized in that the tension cables (5, 6) are formed by two sections of a continuous cable, the portion (9) of which connecting the two tension cables (5, 6) being embedded at the end (7) of the tip (3) into the plastic material constituting the tip (3).

6. Medical instrument according to any one of claims 1 to 5, characterized in that the shaft (1) and the tip (3) have a central lumen (2) and the portion (9) of the cable embedded into the end (7) of the tip (3) forms a loop surrounding the lumen (2).

7. Medical instrument according to any one of claims 1 to 6, characterized in that the ends of one pair of tension cables (5, 6) located opposite one another are fastened at two points on the adjusting wheel (8) that are symmetrical with respect to the direction of the shaft (1) and the axis of rotation of the adjusting wheel (8).

8. Medical instrument according to any one of claims 1 to 7, characterized in that a side conduit (42) that connects to the lumen (2) is provided on the handle (4).

9. Medical instrument according to any one of claims 1 to 8, characterized in that there are incorporated into the plastic material of the tip (3) strips (43) that are located with one narrow side (44) facing one another, symmetrically and transversely to the axis of the shaft (1), with the narrow side (44) facing the lumen (2), with the strips (43) being in each case displaced by 90 degrees with respect to the tension cables (5, 6).

10. Medical instrument according to any one of claims 1 to 9, characterized in that a braid (45) is incorporated into the plastic material of the shaft (1).

## Revendications

1. Instrument médical, composé d'une tige (1) relativement rigide et d'une pointe flexible (3) disposée à une extrémité de la tige (1), dans le prolongement de cette dernière, et à l'extrémité externe (7) de laquelle on peut fixer des câbles tracteurs (5, 6) dont chacun est guidé juste en dessous des surfaces de la tige (1) et de la pointe (3) de façon sensiblement parallèle à la pointe (3) et à la tige (1) jusqu'à l'autre extrémité de cette dernière dans un canal tubulaire et qui sont reliés à l'autre extrémité de la tige (1) par un organe de manoeuvre qui permet un raccourcissement d'un câble tracteur (5, 6) pour conférer à la pointe (3) une courbure sélectionnable dans la direction du câble tracteur raccourci (5, 6), et dans lequel la tige (1) et la pointe (3) sont composées de pièces de matière plastique extrudées qui sont réunies à la jonction (10) entre la tige (1) et la pointe (3), l'autre extrémité de la tige (1) étant fixée dans une poignée (4), caractérisé en ce qu'au niveau de la poignée (4) les canaux de la tige (1) guidant les câbles tracteurs (5, 6) sont ouverts vers l'extérieur, qu'une molette (8) dépassant des deux côtes, de la poignée (4) en tant qu'organe de manoeuvre repose sur paliers de façon à pouvoir pivoter autour d'un axe de rotation perpendiculaire à l'axe du tronçon de la tige (1) fixé dans la poignée (4), les extrémités des câbles tracteurs (5, 6) sont fixés à la molette (8) et la molette (8) repose à sa périphérie dans un évidement de la poignée (4) et présente un évidement central (23) à travers lequel on peut faire passer le tronçon de la tige (1) fixé dans la poignée (4).

2. Instrument médical selon la revendication 1, caractérisé en ce que la molette (8) se compose de deux pièces (15, 16) en forme de disque qui présentent sur leurs faces tournées l'une vers l'autre des creux et des bosses (17 à 22) qui forment l'évidement destiné à la tige (1) et les surfaces de guidage destinées à l'extrémité du câble tracteur (5, 6) sortant de la tige et en ce que l'extrémité du câble tracteur (5, 6) est pincée entre deux parties superficielles accolées (17 à 19) des pièces en forme de disque (15, 16).

3. Instrument médical selon la revendication 1 ou 2, caractérisé en ce que les câbles tracteurs (5, 6), au niveau de la tige (1), à l'intérieur du canal, sont enrobés d'une gaine (41).

4. Instrument médical selon les revendications 1 à 3, caractérisé en ce qu'est disposée à l'extrémité externe (7) de la pointe (3) une bague à laquelle sont fixés les câbles tracteurs (5, 6).

5. Instrument médical selon une des revendications 1 à 4, caractérisé en ce que les câbles tracteurs (5, 6) sont formés de deux tronçons d'un câble continu dont le fragment (9) réunissant les deux câbles (5, 6) est scellé par fusion à l'extrémité (7) de la pointe (3) dans la matière plastique formant la pointe (3).

6. Instrument médical selon une des revendications 1 à 5, caractérisé en ce que la tige (1) et la pointe (3) présentent une lumière centrale et en ce que le fragment (9) de câble scellé par fusion dans l'extrémité (7) de la pointe (3) forme une boucle entourant la lumière (2).

7. Instrument médical selon une des revendications 1 à 6, caractérisé en ce que les extrémités d'une paire de câbles tracteurs (5, 6) opposés sont fixées à deux emplacements de la molette (8) symétriques par rapport à la direction de la tige (1) et à l'axe de rotation de la molette (8).

8. Instrument médical selon une des revendications 1 à 7, caractérisé en ce qu'il est prévu sur la poignée (4) un canal latéral (42) qui communique avec la lumière (2).

9. Instrument médical selon une des revendications 1 à 8, caractérisé en ce que sont incorporées dans la matière plastique de la pointe (3) des bandes qui sont opposées par un côté étroit (44) symétriquement et perpendiculairement à l'axe de la tige (1) et qui sont orientées par leur côté étroit (44) en direction de la lumière (22), les bandes (43) étant décalées respectivement de 90° par rapport aux câbles tracteurs (5, 6).

10. Instrument médical selon une des revendications 1 à 9, caractérisé en ce qu'une gaine tressée (45) est incorporée dans la matière plastique de la tige (1).
